# EUROPÄISCHE PATENTSCHRIFT

(11) **EP 0 763 517 B1**
(45) Veröffentlichungstag und Bekanntmachung des Hinweises auf die Patenterteilung: **08.12.1999**
(21) Anmeldenummer: 96114445.8
(22) Anmeldetag: 10.09.1996
(51) Int. Cl.: C07C 67/44, C07C 69/675

(54) **Verfahren zur Herstellung von Hydroxypivalinsäureneopentylglykolester**
Process for the preparation of the neopentylglycol ester of hydroxypivalic acid
Procédé de préparation de l'ester du néopentylglycol et de l'acide hydroxypivalique

(30) Priorität: 18.09.1995 DE 19534496
(43) Veröffentlichungstag der Anmeldung: 19.03.1997
(73) Patentinhaber: BASF AKTIENGESELLSCHAFT, 67056 Ludwigshafen (DE)
(72) Erfinder: Wittwer, Arnold, Dr., 67434 Neustadt (DE); Sauerwald, Manfred, Dr., 67149 Meckenheim (DE)

(56) Entgegenhaltungen:
- FR-A- 2 069 597
- US-A- 3 057 911

## Beschreibung

Die vorliegende Erfindung betrifft ein verbessertes Verfahren zur Herstellung von Hydroxypivalinsäureneopentylglykolester (HPN) aus einer wäßrigen, 80 bis 98 Gew.-% Hydroxypivalinaldehyd (HPA) enthaltenden Lösung.

HPN kann durch die Tischtschenko-Reaktion aus 2 Äquivalenten HPA hergestellt werden.

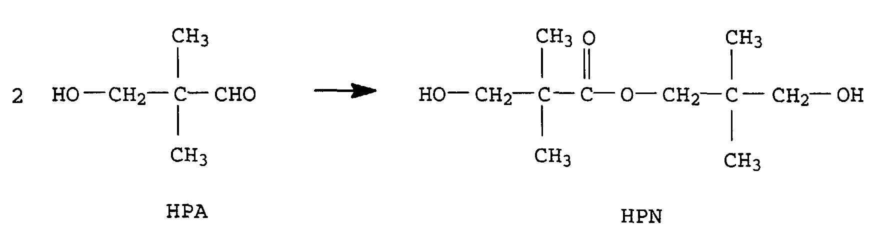

HPN ist ein wertvoller Ausgangsstoff für die Herstellung von Polyestern, Kunstharzen und Weichmachern.

Die Reaktion erfolgt schon durch Erhitzen einer wäßrigen HPA-Lösung auf über 80°C. Der Umsatz ist aber auch bei langer Verweilzeit unbefriedigend. Bei Erhöhung der Reaktionstemperatur sinkt die Selektivität der Umsetzung zu HPN so weit ab, daß die Reaktion aus wirtschaftlichen Gründen unattraktiv ist (DE-A 11 68 411).

HPA kann weiterhin in Gegenwart von basischen Katalysatoren wie Ca(OH)₂ oder CaO hergestellt werden (DE-A 16 43 650, DE-A 22 34 110). Durch die Dosierung des festen Katalysators und insbesondere durch die Abtrennung der Katalysatoren kommt es jedoch in großtechnischen Verfahren zu Problemen. Diese werden zum Teil dadurch gelöst, daß man bei Verwendung von Ca(OH)₂ als Katalysator eine Aufarbeitung mit Ameisensäure vornimmt, welche zur Ausfällung von Calciumformiat führt (DE-A 22 34 358). Eine kontinuierliche Fahrweise ist jedoch mit dem beschriebenen Verfahren technisch problematisch. Weiterhin kann die zur Fällung des Katalysators verwendete Ameisensäure zu Korrosionsproblemen führen, wenn sie nicht quantitativ aus der Produktlösung abgetrennt wird.

Es bestand daher die Aufgabe, ein Verfahren bereitzustellen, das die genannten Nachteile vermeidet. Insbesondere bestand die Aufgabe darin, ein Verfahren zu finden, das eine technisch einfach zu realisierende kontinuierliche Herstellung von HPN aus HPA erlaubt.

Demgemäß wurde ein Verfahren zur Herstellung von HPN aus einer wäßrigen, 80 bis 98 Gew.-% HPA enthaltenden Lösung gefunden, das dadurch gekennzeichnet ist, daß man die wäßrige, 80 bis 98 Gew.-% HPA enthaltende Lösung in einer Reaktionskolonne bei einem Druck von 20 bis 200 mbar und einer Kolonnenkopftemperatur von 80 bis 150°C umsetzt, ein Gemisch aus leichtsiedenden Komponenten über Kopf abdestilliert und Hydroxypivalinsäureneopentylglykolester aus dem Kolonnensumpf abzieht.

Als Ausgangsstoff im erfindungsgemäßen Verfahren wird eine wäßrige, 80 bis 98 Gew.-% HPA enthaltende Lösung eingesetzt. Im allgemeinen enthält die Lösung weniger als 15 Gew.-%, bevorzugt weniger als 10 Gew.-% und besonders bevorzugt weniger als 5 Gew.-% Wasser. Der HPA-Gehalt beträgt bevorzugt 85 bis 98 Gew.-%.

In einer bevorzugten Ausführungsform wird HPA durch Umsetzung von Isobutyraldehyd und wäßriger Formaldehydlösung in Gegenwart katalytischer Mengen Trialkylamin hergestellt (EP-A 173 976). Je nach dem Wassergehalt der so erhaltenen Produktlösung kann diese direkt oder nach Entwässerung, die bevorzugt destillativ erfolgt, im erfindungsgemäßen Verfahren eingesetzt werden.

Bedingt durch ihre Herstellweise kann die wäßrige HPA-Lösung als Nebenbestandteile z.B. Neopentylglykol (NPG) und HPN sowie Ammoniumsalze enthalten.

Die wäßrige, 80 bis 98 Gew.-% HPA enthaltende Lösung wird in eine Reaktionskolonne gegeben. Diese Reaktionskolonne ist bevorzugt eine Destillationskolonne, jedoch kommen auch solche Reaktorformen in Betracht, aus denen während der Reaktion über Kopf abdestilliert und das Produkt aus dem Sumpf abgezogen werden kann.

Bevorzugt wird die Ausgangslösung im mittleren Drittel der Reaktionskolonne eingespeist. Die Reaktionskolonne enthält in einer bevorzugten Ausführungsform im Verstärkungsteil, bevorzugt in den oberen beiden Dritteln, Einbauten wie Ventilböden, besonders bevorzugt Glockenböden. Weiterhin ist es bevorzugt, im Abtriebsteil, bevorzugt im unteren Drittel der Reaktionskolonne Füllkörper zu verwenden, d.h. Füllungen aus inerten Materialien, z.B. Metallpallringe, Keramikpallringe oder Glaskörper.

Die Reaktionskolonne wird unter vermindertem Druck von 10 bis 200 mbar, bevorzugt 30 bis 150 mbar und besonders bevorzugt 40 bis 120 mbar betrieben. Die Kolonnenkopftemperatur beträgt 80 bis 150°C, bevorzugt 90 bis 120°C und besonders bevorzugt 95 bis 105°C, wobei es sich als vorteilhaft erwiesen hat, die Kolonnenkopftemperatur entsprechend der Siedetemperatur des HPA unter dem jeweiligen Reaktionsdruck zu wählen.

Die Verweilzeit kann 1 bis 10 h, bevorzugt 3 bis 5 h betragen. Sie kann über die am Kopf bzw. im Sumpf entnommene Menge an Reaktionsprodukten gesteuert werden.

Am Kolonnenkopf destilliert ein Gemisch ab, das im wesentlichen Wasser, Neopentylglykol sowie nicht umgesetzten Hydroxypivalinaldehyd enthält. Dieses kann nach Kondensation und gegebenenfalls weiteren Destillationsschritten aufgearbeitet werden, wobei HPA in die Reaktionskolonne zurückgeführt werden kann und Neopentylglykol als Wertprodukt anfällt.

HPN wird aus dem Sumpf der Reaktionskolonne abgezogen. Das Rohprodukt weist in der Regel eine hohe Reinheit auf. Als Nebenprodukt fallen vor allem HPA und NPG an, die erforderlichenfalls leicht destillativ abgetrennt werden können. Auch hier hat sich eine Rückführung des so erhaltenen HPA in die Reaktionskolonne als vorteilhaft erwiesen.

Das erfindungsgemäße Verfahren läßt sich in technisch einfacher Weise kontinuierlich betreiben. Es überführt HPA in hoher Selektivität und Ausbeute in HPN. Das erhaltene Rohprodukt ist - wenn erforderlich - in einfacher Weise von Nebenprodukten zu befreien.

### Beispiele

Die Herstellung der eingesetzten wäßrigen HPA-Lösung erfolgte gemäß dem in DE-A 19 57 591 beschriebenen Verfahren.

### Beispiel 1

Stündlich wurden aus 200 g einer Lösung, die im wesentlichen 134 g HPA, 2,4 g NPG, 3 g HPN und 44,6 g Wasser enthielt, bei 100°C und 200 mbar Druck leichtsiedende Verbindungen wie Methanol, Isobutanol, Isobutyraldehyd, Formaldehyd und Wasser abdestilliert. Die verbleibende Lösung (0,5 Gew.-% Wasser) wurde standgeregelt in den mittleren Teil einer Reaktionskolonne gegeben, die aus 15 Glockenböden und einer als Abtriebsteil dienenden Füllkörperkolonne bestand, die Metallpallringe enthielt. Am Kolonnenkopf wurden kontinuierlich Leichtsieder abgezogen. Bei einem Druck von 80 mbar und 105°C Kolonnenkopftemperatur wurden bei einer Verweilzeit von 4 h stündlich aus dem Sumpf 139 g Rohprodukt abgezogen, das nach gaschromatographischer Analyse 84 Gew.-% HPN, 4,9 Gew.-% NPG und < 0,1 Gew.-% HPA enthielt.

### Beispiel 2

Analog zu Beispiel 1 wurden stündlich 200 g einer Lösung behandelt, die im wesentlichen 124 g HPA, 2,3 g NPG, 4,0 g HPN und 44,6 g Wasser enthielt. Die in die Reaktionskolonne eingegebene Lösung enthielt 0,7 Gew.-% Wasser. Bei 50 mbar und einer Kolonnenkopftemperatur von 99°C wurden bei einer Verweilzeit von 4 h stündlich 130 g Rohprodukt aus dem Sumpf entnommen, das 82 Gew.-% HPN, 5,7 Gew.-% NPG und 0,5 Gew.-% HPA enthielt.

### Beispiel 3

Analog zu Beispiel 1 wurden stündlich 200 g einer Lösung behandelt, die im wesentlichen 123 g HPA, 1,9 g NPG, 3,1 g HPN und 45,4 g Wasser enthielt. Die in die Reaktionskolonne eingegebene Lösung enthielt 1,0 Gew.-% Wasser. Bei 30 mbar und einer Kolonnenkopftemperatur von 103°C wurden bei einer Verweilzeit von 4 h stündlich 128 g Rohprodukt aus dem Sumpf entnommen, das 73,5 Gew.-% HPN, 5,1 Gew.-% NPG und 10,9 Gew.-% HPA enthielt.

### Beispiel 4

Analog zu Beispiel 1 wurden stündlich 200 g einer Lösung behandelt, die im wesentlichen 126 g HPA, 1,4 g NPG, 3,3 g HPN und 46,6 g Wasser enthielt. Die in die Reaktionskolonne gegebene Lösung enthielt 1,5 Gew.-% Wasser. Bei 150 mbar und einer Kolonnenkopftemperatur von 114°C wurden bei einer Verweilzeit von 4 h stündlich 130 g Rohprodukt aus dem Sumpf entnommen, das 77 Gew.-% HPN, 6,5 Gew.-% NPG und 0,8 Gew.-% HPA enthielt.

### Beispiel 5

Analog zu Beispiel 1 wurden stündlich 250 g einer Lösung behandelt, die im wesentlichen 188 g HPA, 3,1 g NPG, 2,8 g HPN und 38 g Wasser enthielt. Die in die Reaktionskolonne gegebene Lösung enthielt 0,2 Gew.-% Wasser. Bei 60 mbar und einer Kolonnenkopftemperatur von 103°C wurden bei einer Verweilzeit von 3 h stündlich 194 g Rohprodukt aus dem Sumpf entnommen, das 79 Gew.-% HPN, 3,9 Gew.-% NPG und 11,1 Gew.-% HPA enthielt.

## Patentansprüche

1. Verfahren zur Herstellung von Hydroxypivalinsäureneopentylglykolester aus einer wäßrigen, Hydroxypivalinaldehyd enthaltenden Lösung, dadurch gekennzeichnet, daß man die wäßrige, 80 bis 98 Gew.-% Hydroxypivalinaldehyd enthaltende Lösung in einer Reaktionskolonne bei einem Druck von 20 bis 200 mbar und einer Kolonnenkopftemperatur von 80 bis 150°C umsetzt, ein Gemisch aus leichtsiedenden Komponenten über Kopf abdestilliert und Hydroxypivalinsäureneopentylglykolester aus dem Kolonnensumpf abzieht.

2. Verfahren nach Anspruch 1, dadurch gekennzeichnet, daß man die wäßrige, 80 bis 98 Gew.-% Hydroxypivalinaldehyd enthaltende Lösung im mittleren Drittel der Reaktionskolonne einspeist.

3. Verfahren nach Anspruch 1 oder 2, dadurch gekennzeichnet, daß man eine im Verstärkungsteil mit Glockenböden versehene Kolonne als Reaktionskolonne verwendet.

4. Verfahren nach den Ansprüchen 1 bis 3, dadurch gekennzeichnet, daß man eine im Abtriebsteil mit Füllkörpern versehene Kolonne als Reaktionskolonne verwendet.

5. Verfahren nach den Ansprüchen 1 bis 4, dadurch gekennzeichnet, daß die wäßrige, Hydroxypivalinaldehyd enthaltende Lösung weniger als 15 Gew.-% Wasser enthält.

6. Verfahren nach den Ansprüchen 1 bis 5, dadurch gekennzeichnet, daß man eine wäßrige, 80 bis 98 Gew.-% Hydroxypivalinaldehyd enthaltende Lösung verwendet, die durch Umsetzung von Isobutyraldehyd und wäßriger Formaldehydlösung in Gegenwart katalytischer Mengen an Trialkylamin hergestellt und gegebenenfalls entwässert wurde.

7. Verfahren nach den Ansprüchen 1 bis 6, dadurch gekennzeichnet, daß die Kolonnenkopftemperatur 90 bis 120°C beträgt.

## Claims

1. A process for preparing neopentyl glycol hydroxypivalate from an aqueous hydroxypivalaldehyde-containing solution, which comprises reacting the aqueous solution containing from 80 to 98% by weight of hydroxypivalaldehyde in a reaction column under a pressure of from 20 to 200 mbar and at a temperature at the head of the column of from 80 to 150°C, distilling off a mixture of low-boiling components overhead, and taking off hydroxypivalic acid neopentyl glycol ester from the bottom of the column.

2. A process as claimed in claim 1, wherein the aqueous solution containing from 80 to 98% by weight of hydroxypivalaldehyde is fed into the middle third of the reaction column.

3. A process as claimed in claim 1 or 2, wherein a column provided with bubble trays in the enrichment section is used as reaction column.

4. A process as claimed in claims 1 to 3, wherein a column provided with packings in the stripping section is used as reaction column.

5. A process as claimed in claims 1 to 4, wherein the aqueous hydroxypivalaldehyde-containing solution contains less than 15% by weight of water.

6. A process as claimed in claims 1 to 5, wherein an aqueous solution which contains from 80 to 98% by weight of hydroxypivalaldehyde and has been prepared by reacting isobutyraldehyde and aqueous formaldehyde solution in the presence of catalytic amounts of trialkylamine, and from which water has been removed where appropriate, is used.

7. A process as claimed in claims 1 to 6, wherein the temperature at the top of the column is from 90 to 120°C.

## Revendications

1. Procédé de préparation d'ester néopentylglycolique d'acide hydroxypivalique à partir d'une solution aqueuse contenant l'aldéhyde hydroxypivalique, caractérisé en ce que l'on fait réagir la solution aqueuse contenant 80-98% en poids d'aldéhyde hydroxypivalique dans une colonne de réaction, sous une pression de 20-200 mbar et avec une température en tête de colonne de 80-150°C, on élimine en tête par distillation un mélange de composants à bas points d'ébullition et on extrait du fond de colonne l'ester néopentylglycolique d'acide hydroxypivalique.

2. Procédé selon la revendication 1, caractérisé en ce que la solution aqueuse contenant 80-98% en poids d'aldéhyde hydroxypivalique est alimentée dans le tiers du milieu de la colonne de réaction.

3. Procédé selon la revendication 1 ou 2, caractérisé en ce que l'on utilise, en tant que colonne de réaction, une colonne comportant des plateaux à cloches dans la zone de concentration.

4. Procédé selon l'une quelconque des revendications 1 à 3, caractérisé en ce que l'on utilise, en tant que colonne de réaction, une colonne comportant des matières de garnissage dans la zone d'épuisement.

5. Procédé selon l'une quelconque des revendications 1 à 4, caractérisé en ce que la solution aqueuse contenant l'aldéhyde hydroxypivalique contient moins de 15% en poids d'eau.

6. Procédé selon l'une quelconque des revendications 1 à 5, caractérisé en ce que l'on utilise une solution aqueuse contenant 80-98% en poids d'aldéhyde hydroxypivalique produite par réaction d'isobutyraldéhyde et d'une solution aqueuse de formaldéhyde, en présence de quantités catalytiques de trialkylamine et éventuellement débarrassée de l'eau.

7. Procédé selon l'une quelconque des revendications 1 à 6, caractérisé en ce que la température en tête de colonne s'élève à 90-120°C.
